Europäisches Patentamt

**European Patent Office**

**Office européen des brevets**

(19)

(11) Veröffentlichungsnummer : **0 025 868**
**B1**

(12)

# EUROPÄISCHE PATENTSCHRIFT

(45) Veröffentlichungstag der Patentschrift :
**22.09.82**

(21) Anmeldenummer : **80104879.4**

(22) Anmeldetag : **16.08.80**

(51) Int. Cl.³ : **C 07 C103/52**, C 07 G 15/00,
A 61 K 37/26, B 01 D 9/02

(54) **Insulinkristallsuspension und Verfahren zu ihrer Herstellung.**

(30) Priorität : **22.08.79 DE 2933946**

(43) Veröffentlichungstag der Anmeldung :
**01.04.81 (Patentblatt 81/13)**

(45) Bekanntmachung des Hinweises auf die Patenterteilung : **22.09.82 Patentblatt 82/38**

(84) Benannte Vertragsstaaten :
**AT BE CH DE FR GB IT LI NL SE**

(56) Entgegenhaltungen :
**Chemical Abstracts Band 85, Nr. 11,
13 September 1976 Columbus, Ohio, USA
G. BENTLEY et al. « Structure of insulin
in 4-zinc insulin » Seite 181, Spalte 2,
Abstract Nr. 73726f
Chemical Abstracts Band 90, Nr. 15, 09 April 1979
Columbus, Ohio, USA G. BENTLEY et al.
« Rhombohedral insulin crystal transformation »
Seite 211, Spalte 2, Abstract Nr. 116664g**

(73) Patentinhaber : **HOECHST AKTIENGESELLSCHAFT
Postfach 80 03 20
D-6230 Frankfurt/Main 80 (DE)**

(72) Erfinder : **Thurow, Horst, Dr.
Parkstrasse 20
D-6233 Kelkheim (Taunus) (DE)**

Imprimerie Jouve, 18, rue St-Denis, 75001 Paris, France

EP 0 025 868 B1

Insulinkristallsuspension und Verfahren zu ihrer Herstellung

Die Erfindung betrifft Insulinpräparate, die Insulin-Zink-Kristalle, insbesondere solche aus Schweine-Insulin enthalten. Diese Kristalle sind scharfkantige Rhomboeder mit 2-Zn-Struktur. Sie werden durch Strukturtransformation aus Rhomboedern mit 4-Zn-Struktur erhalten.

Es ist bekannt, Insulin aus wässrigen Medien unter Zusatz von zweiwertigen Metallen, vorzugsweise Zink, und einer Puffersubstanz wie Ammoniumacetat oder Natriumacetat bei einem pH-Wert zwischen 5 und 7 als Rhomboeder zu kristallisieren. In manchen Fällen wird zur Vermeidung einer Ausfällung von amorphem Insulin ein organisches Lösungsmittel wie Aceton zugesetzt. Außerdem kann es zweckmäßig sein, den Kristallisationsprozeß durch Zugabe von Impfkristallen zu beeinflussen. Bei Anwesenheit von Halogeniden (Chlorid oder Jodid), Harnstoff oder Formamid in Kristallisationsmedium, entstehen, in Abhängigkeit von deren Konzentration, Rhomboeder verschiedener Kristallstruktur. So entstehen bei einer Konzentration bis zu 6 % Natriumchlorid im Kristallisationsmedium Rhomboeder mit sogenannter 2-Zn-Struktur und bei einem höheren Natriumchloridgehalt Rhomboeder mit sogenannter 4-Zn-Struktur (M. J. Adams et al., Nature *224*, 491 (1969) und G. Bentley et al., Nature *261*, 166 (1976)). Beide Kristallformen nehmen mit steigendem pH-Wert des umgebenden Mediums zusätzlich Zink auf. Der Brutto-Zinkgehalt in den Kristallen und die Zinkkonzentration im umgebenden Medium sowie Größe und Form der Kristalle bestimmen die Lösungsgeschwindigkeit der Kristalle. So haben z.B. Kristalle aus Schweineinsulin mit einem Zinkgehalt von 0,8 bis 2,5 %, bezogen auf das Insulingewicht, in neutraler Lösung eine verzögerte therapeutische Wirkung.

Es ist ferner bekannt, daß Kristallisation unter den Bedingungen, die zu Rhomboedern mit 2-Zn-Struktur führen, schlecht ausgebildete Kristalle liefert. Oft enstehen dabei abgerundete Kristallkörper, deformierte Rhomboeder oder Zwillingsformen (US-PS 2 920 014). Unter den Bedingungen, die zu Rhomboedern mit 4-Zn-Struktur führen, entstehen hingegen gut ausgebildete scharfkantige Rhomboeder. Es ist leicht einzusehen, daß gleichmäßig ausgebildete Kristalle in Insulinpräparaten für therapeutische Zwecke von Vorteil sind, insbesondere weil eine reproduzierbare Auflösungsgeschwindigkeit der Kristalle ein reproduzierbares Zeit-Wirkungsprofil des Präparates gewährleistet.

Es hat sich gezeigt, daß Insulinkristalle mit 4-Zn-Struktur unter den Bedingungen, wie sie in Insulinpräparaten für therapeutische Zwecke notwendig sind, d.h. bei einem Natriumchlorid-Gehalt bis zu 1,5 %, nicht stabil sind. Dies wirkt sich so aus, daß die Kristalle, die in einem neutralen für therapeutische Zwecke üblichen Medium suspendiert sind und weniger als 1,5 % Zink enthalten, nach einiger Zeit teilweise in Lösung gehen. Enthalten die Kristalle einen höheren Zinkanteil, so zeigen sie nicht reproduzierbare Wirkungseffekte.

Es ist bekannt, daß man 4-Zn-Kristalle aus Schweineinsulin in 2-Zn-Kristalle transformieren kann, wenn man die 4-Zn-Kristalle in ein Medium gibt, welches frei von Natriumchlorid ist und einen pH-Wert von 6,5 hat. Dabei kommt es jedoch zu solch großen Spannungen im Kristall, daß die Transformation mit einer völligen Zerstörung der äußeren Kristallform einhergeht (G. Bentley et al., J. Mol. Biol. *126*, 871 (1978)).

Es wurde nun überraschenderweise gefunden, daß man rhomboederische Insulinkristalle mit 4-Zn-Struktur, und zwar sowohl solche aus Schweineinsulin als auch aus Rinderinsulin, in Kristalle mit 2-Zn-Struktur transformieren kann ohne erkennbare Beeinträchtigung der äußeren Form, so daß man diese transformierten Kristalle zur Herstellung von stabilen Insulinpräparaten für therapeutische Zwecke verwenden kann.

Gegenstand der Erfindung ist demnach ein Verfahren zur Strukturtransformation von rhomboederisch kristallisiertem Insulin, vorzugsweise vom Schwein, mit 4-Zn-Struktur in Rhomboeder mit 2-Zn-Struktur, dadurch gekennzeichnet, daß man die Kristalle mit 4-Zn-Struktur in einem wässrigen Medium, das weniger als 6 % Natriumchlorid enthält und dessen pH-Wert auf 4,5 bis 6,0 eingestellt und durch Zusatz eines Puffers stabilgehalten ist, solange bei einer Temperatur zwischen Raumtemperatur und 45 °C hält, bis in einer Probe keine Kristalle der 4-Zn-Struktur mehr feststellbar sind.

Die transformierten Kristalle haben scharfe Kanten und Ecken sowie glatte Flächen. Sie zeigen die gleiche Transparenz wie die ursprünglichen 4-Zn-Kristalle. Ein Vergleich der Röntgenspektren der Kristalle vor und nach der Transformation zeigt, daß sich die Struktur geändert hat, erkennbar an der Änderung der Beugungswinkel. Unverändert ist aber der Ordnungsgrad im Kristall, erkennbar am Verhältnis von Höhe zur Breite der Peaks.

Zur Herstellung einer Suspension von Insulinkristallen mit 2-Zn-Struktur für therapeutische Zwecke gemäß der Erfindung geht man vom kristallisierten Insulin der 4-Zn-Struktur aus, das vorteilhaft unter sterilen Bedingungen in an sich bekannter Weise kristallisiert wurde. Zur Durchführung der Strukturtransformation der Kristalle wird das umgebende Medium durch eine Lösung ersetzt, die weniger als 6 % (Gew./Vol.) Natriumchlorid, vorzugsweise 0,5-2 % und ein Salz zur Pufferung des pH-Wertes, vorzugsweise Natriumacetat enthält. Die Lösung wird auf einen pH-Wert nahe dem isoelektrischen Punkt von Insulin eingestellt, d.h. zwischen pH 4,5 und 6,0, vorzugsweise zwischen 5,0 und 5,8. Die Strukturtransformation erfolgt bei Raumtemperatur oder bei erhöhter Temperatur bis 45 °C, vorzugsweise 37 °C. Es ist vorteilhaft, aber nicht notwendig, die Suspension während der Umlagerungsreaktion in Bewegung zu halten, beispielsweise durch Rühren oder Schütteln. Die Strukturtransformation der Kristalle läßt sich leicht feststellen, indem man eine Probe entnimmt und, beispielsweise durch Röntgenstrukturanalyse, prüft, ob sich noch Kristalle mit der ursprünglichen 4-Zn-Struktur in der Suspension befinden. Das ist in der Regel, je nach den Reaktionsbedingungen, in einigen Stunden bis Tagen der Fall. Die Geschwindigkeit der Transformation fällt mit steigendem pH-Wert und mit steigender Konzentration an Natriumchlorid im Medium ab. Mit steigender Temperatur nimmt sie zu.

Aromatische Substanzen, wie sie die gebräuchlichen Konservierungsmittel darstellen, hemmen die Transformation. So verhindert z.B. ein Zusatz von 0,1 % Methylparaben die Transformation von Kristallen aus Schweineinsulin. Sie sind daher bei der erfindungsgemäßen Umlagerungsreaktion auszuschließen. Nach Abschluß der Umwandlung wird die Kristallsuspension mit einer Verdünnunglösung verdünnt, so daß sich eine Suspension mit 40, 80 oder 100 I.E./ml Insulinkristallen ergibt. Die Verdünnungslösung enthält vorteilhaft zur Einstellung der Isotonie eine physiologisch verträgliche Substanz, z.B. Natriumchlorid ; zur Pufferung des pH-Wertes ein Puffersalz, z.B. Natriumacetat ; ein Konservierungsmittel, z.B. Methylparaben. Sie wird vorzugsweise mit Natronlauge, auf einen solchen pH-Wert eingestellt, daß sich nach dem Verdünnen in der Kristallsuspension ein pH-Wert im Bereich von 6-8, vorzugsweise pH 6,7-7,5 ergibt. Außerdem ist es notwendig, der Verdünnungslösung soviel Zink zuzusetzen, daß die Insulinkristalle in der neutralen Suspension einen Zinkgehalt von 0,9-2,5 % zeigen.

Die so erhaltene Suspension von stabilen Insulinkristallen mit 2-Zn-Struktur kann man gewünschtenfalls mit einer schnell wirkenden neutralen Lösung von Insulin oder Insulinderivaten oder mit einer verzögert wirkenden neutralen Suspension von amorphem Insulin oder Insulinderivaten mischen, wobei die Stabilität des Komponentenverhältnisses nur vom Zinkgehalt der Mischung abhängt.

Beispiel 1

A 1. Herstellung einer Suspension von Kristallen mit 4-Zn-Struktur

Kristallines Insulin vom Schwein (450 000 I.E.), das 0,5 Gewichtsprozent Zink enthielt, wurde in 400 ml 0,03 n Salzsäure gelöst. Die Lösung wurde mit 15 ml einer 1%igen Zinkchlorid-Lösung in 0,03 n Salzsäure versetzt und mit 0,03 n Salzsäure auf 500 ml ergänzt. Die Lösung wurde sterilfiltriert und mit 500 ml einer ebenfalls sterilfiltrierten Lösung von 70 g Natriumchlorid, 14 g Natriumacetat · 3H$_2$O und 10 ml 1 n Natronlauge in Wasser gemischt. Die Mischung wurde auf pH 5,4-5,5 eingestellt und 48 Stunden bei Raumtemperatur gerührt wobei das Insulin in Rhomboedern mit 4-Zn-Struktur kristallisierte.

A 2. Strukturtransformation von 4-Zn- in 2-Zn-Kristalle

Aus 1 l Kristallsuspension, hergestellt nach A 1 wurden nach Absetzen der Kristalle aus dem klaren überstand 888 ml unter sterilen Bedingungen abgepumpt und durch 1 013 ml einer sterilfiltrierten Lösung ersetzt, die 50 mg Zinkchlorid und 1,125 g Natriumchlorid enthielt und mit 1 n Salzsäure auf pH 5,4-5,5 eingestellt war. Die Kristallsuspension wurde anschließend 7 Tage bei Raumtemperatur und danach 5 Tage bei 37 °C gerührt. Das Röntgenspektrum zeigte danach eine vollständige Umwandlung der 4-Zn- in die 2-Zn-Struktur.

A 3. Herstellung einer neutralen Kristallsuspension mit 40 I.E./ml

1,125 ml der nach A 1 und A 2 hergestellten Kristallsuspension wurden mit 10,125 l einer sterilfiltrierten Lösung versetzt, die 81 g Natriumchlorid, 14,175 g Natriumacetat · 3H$_2$O, 0,8-1,1 g Zinkchlorid, 11,250 g Methylparaben und soviel 1 n Natronlauge enthielt, daß sich in der Mischung ein pH-Wert im Bereich von 6,9 bis 7,4 einstellte.

Beispiel 2

Herstellung eines zweiphasigen Präparates aus 70 % Kristallen aus Schweineinsulin und 30 % amorphen Des-B1-Phenylalanininsulin vom Schwein.

1,125 l der nach A 1 und A 2 (Beispiel 1) hergestellten Kristallsuspension wurden mit 14,946 l einer sterilen Suspension versetzt, die 192 900 I.E. amorphes Des-B1-Phenylalanin-Insulin vom Schwein, 119,6 g Natriumchlorid, 20,9 g Natriumacetat · 3H$_2$O, 2,362 g Zinkchlorid, 16,07 g Methylparaben und soviel 1 n Natronlauge enthielt, daß sich in der Mischung ein pH-Wert im Bereich von 7,2-7,5 einstellte.

Beispiel 3

Herstellung eines zweiphasigen Präparates aus 75 % Kristallen aus Schweine-Insulin und 25 % gelöstem Des-B1-Phenylalanin-Insulin vom Schwein.

1,121 5 l der nach A 1 und A 2 (Beispiel 1) hergestellten Kristallsuspension wurden mit 13,875 l einer sterilen Lösung versetzt, die 150 000 I.E. Des-B1-Phenylalanin-Insulin vom Schwein, 111,04 g Natriumchlorid, 19,43 g Natriumacetat · 3H$_2$O, 292 mg Zinkchlorid, 15,00 g Methylparaben und soviel 1 n Natronlauge enthielt, daß sich in der Mischung ein pH-Wert von 6,8-7,0 einstellte.

Beispiel 4

Herstellung eines zweiphasigen Präparates aus 75 % Kristallen aus Schweine-Insulin, 12,5 % gelöstem Schweine-Insulin und 12,5 % gelöstem

Des-B1-Phenylalanin-Insulin vom Schwein.

1,125 l der nach A 1 und A 2 (Beispiel 1) hergestellten Kristallsuspension wurden mit 13,875 l einer sterilen Lösung versetzt, die 75 000 I.E. Insulin vom Schwein und 75 000 I.E. Des-B1-Phenylalanin-Insulin vom Schwein, 111,04 g Natriumchlorid, 19,43 g Natriumacetat · 3H$_2$O, 354 mg Zinkchlorid, 15,00 g Methylparaben und soviel 1 n Natronlauge enthielt, daß sich in der Mischung ein pH-Wert von 6,8-7,0 einstellte.

**Ansprüche**

1. Verfahren zur Strukturtransformation von rhomboedrisch kristallisiertem Insulin, vorzugsweise vom Schwein, mit 4-Zn-Struktur in Rhomboeder mit 2-Zn-Struktur, dadurch gekennzeichnet, daß man die Kristalle mit 4-Zn-Struktur in einem wässrigen Medium, das weniger als 6 % Natriumchlorid enthält und dessen pH-Wert auf 4,5 bis 6,0 eingestellt und durch Zusatz eines Puffers stabilgehalten ist, solange bei einer Temperatur zwischen Raumtemperatur und 45 °C hält, bis in einer Probe keine Kristalle der 4-Zn-Struktur mehr feststellbar sind.

2. Verzögert wirkende Kristallsuspension aus Insulin, vorzugsweise vom Schwein, in einem wässrigen Medium, dadurch gekennzeichnet, daß sie Insulin-Kristalle in Form von Rhomboedern mit 2-Zn-Struktur enthält, welche durch Strukturformation aus Rhomboedern mit 4-Zn-Struktur erhalten wurden.

**Claims**

1. A process for the structural transformation of rhombohedrally crystallized insulin, preferably of pig origin, having a 4-Zn structure to rhombohedrons having a 2-Zn structure, which comprises maintaining the crystals having a 4-Zn structure in an aqueous medium containing less than 6 % of sodium chloride, the pH of which medium is adjusted to 4.5 to 6.0 and maintained stable by addition of a buffer, at a temperature ranging from room temperature to 45 °C until in a sample crystals of the 4-Zn structure are no longer present.

2. Crystal suspension of insulin, preferably pig insulin, in an aqueous medium, having a delayed action, wherein insulin crystals in the form of rhombohedrons of 2-Zn structure are present which have been obtained by structural transformation of rhombohedrons having a 4-Zn structure.

**Revendications**

1. Procédé de transformation structurale d'une insuline, de préférence de porc, cristallisée en rhomboèdres, ayant la structure 4-Zn, en rhomboèdres ayant la structure 2-Zn, caractérisé en ce qu'on maintient les cristaux de structure 4-Zn dans un milieu aqueux contenant moins de 6 % de chlorure de sodium, dont le pH est ajusté à 4,5-6,0 et stabilisé par addition d'un tampon, à une température comprise entre la température ambiante et 45 °C, jusqu'à ce qu'on ne décèle plus de cristaux de la structure 4-Zn dans un échantillon.

2. Suspension cristalline à action retardée d'insuline, de préférence de porc, dans un milieu aqueux, caractérisée en ce qu'elle contient des cristaux d'insuline sous la forme de rhomboèdres ayant la structure 2-Zn, qui ont été obtenus par transformation structurale à partir de rhomboèdres ayant la structure 4-Zn.